# EUROPEAN PATENT SPECIFICATION

(11) **EP 4 005 544 B1**
(45) Date of publication and mention of the grant of the patent: **03.12.2025**
(21) Application number: 20883346.7
(22) Date of filing: 26.10.2020
(51) Int. Cl.: A61F 13/49, A61F 13/494

(54) **ABSORBENT ARTICLE**
ABSORBIERENDER ARTIKEL
ARTICLE ABSORBANT

(30) Priority: 01.11.2019 JP 2019200004
(43) Date of publication of application: 01.06.2022
(73) Proprietor: Kao Corporation, Tokyo 103-8210 (JP)
(72) Inventor: SHIRAKAWA, Takashi, Haga-gun, Tochigi 321-3497 (JP)
(74) Representative: Vossius & Partner Patentanwälte Rechtsanwälte mbB
(86) International application number: PCT/JP2020/040048
(87) International publication number: WO 2021/085361

(56) References cited:
- EP-A1- 2 246 018
- JP-A- 2009 178 274
- JP-A- H0 810 287
- JP-A- H05 293 134
- JP-A- H1 128 224
- US-A1- 2003 120 248

## Description

### Technical Field

The present invention relates to an absorbent article including leak-proof cuffs that rise toward the skin of a wearer.

### Background Art

Absorbent articles such as a disposable diaper include an absorbent article including a lateral leakage prevention member also called a leak-proof cuff, a three-dimensional gathering, a three-dimensional guard, or the like. Typically, a pair of the lateral leakage prevention members are provided on the respective sides along the longitudinal direction of an assembly of an absorbent article (direction coincident with the front-rear direction of a wearer), each include a lateral leakage prevention member forming sheet and elastic members fixed in a stretched state to the sheet, and are to rise toward the skin of a wearer wearing the absorbent article to dam excrements such as feces, thus preventing lateral leakage.

Patent Literatures 1 (JP H11-285510 A) and 2 (JP 2011-050501 A) disclose articles including, as the lateral leakage prevention member, lateral leakage prevention member forming sheets that each include a base portion fixed to another member and a raising portion raising the sheet toward a wearer from the base portion as a starting point. The raising portion is folded along a fold portion extending in the longitudinal direction and includes an inner side extension portion extending inward in the lateral direction (the direction orthogonal to the longitudinal direction) from the base portion to the fold portion and an outer side extension portion extending outward in the lateral direction from the fold portion and to be placed closer to the skin of a wearer than the inner side extension portion. In a lateral leakage prevention member according to Patent Literature 3 (JP 2008-307223 A), a raising portion to rise toward a wearer has a T-shape in a cross section along the lateral direction.

US 2003/0120248 A and EP 2 246 018 A1 disclose a disposable absorbent article having a barrier leg cuff and an elasticized outer leg cuff.

### Summary of Invention

The present invention relates to an absorbent article as defined by claim 1, having a longitudinal direction corresponding to the front-rear direction of a wearer and a lateral direction orthogonal to the longitudinal direction, and the absorbent article includes an absorbent assembly including an absorbent member and a pair of leak-proof cuffs that extend in the longitudinal direction and are provided on a skin-facing surface of the absorbent assembly while a separating portion is interposed therebetween. Each of the pair of leak-proof cuffs includes a leak-proof cuff forming sheet and has a base portion at which the leak-proof cuff forming sheet is fixed to another member and a raising portion that raises the leak-proof cuff forming sheet toward the wearer from the base portion as a starting point. The leak-proof cuff forming sheet in the raising portion is folded along a fold portion extending in the longitudinal direction. The raising portion is sectioned into a first portion from the fold portion to a side edge of the leak-proof cuff forming sheet along the longitudinal direction and a second portion from the fold portion to the base portion and includes a sheet overlapping portion in which the first portion and the second portion facing together are joined through a joined portion. The first portion includes a free edge portion, and the free edge portion is not fixed to any other member, and is an extension portion of the leak-proof cuff forming sheet extending from the joined portion. An extension length of the free edge portion from the joined portion is not less than 0.05 times a length from the joined portion to the base portion in the second portion. Between the first portion and the second portion, a plurality of leak proof cuff forming elastic members are arranged in the lateral direction and an innermost elastic member located innermost in the lateral direction of the plurality of leak proof forming elastic members has a larger stress than an outermost elastic member located outermost in the lateral direction when the absorbent article is worn.

### Brief Description of Drawings

[FIG. 1] FIG. 1 is a partially broken, developed plan view schematically showing a skin-facing surface side (topsheet side) of an open type disposable diaper in a flat-out, uncontracted state as an embodiment of an absorbent article in the present invention.
[FIG. 2] FIG. 2 is a cross-sectional view schematically showing a cross section taken along line I-I in FIG. 1.
[FIG. 3] FIG. 3 is a schematic perspective view of the diaper shown in FIG. 1 in a natural state.
[FIG. 4] FIG. 4 is a cross-sectional view of the diaper in a natural state shown in FIG. 3 (corresponding to FIG. 2).
[FIGS. 5] FIGS. 5 are each a schematic view of one set of joined portions of a pair of leak-proof cuffs observed in the extending direction (longitudinal direction), in the diaper shown in FIG. 1; FIG. 5(a) is a schematic view showing the joined portions observed from laterally outside the joined portions (from the relatively distant side from the center of the diaper in the lateral direction); and FIG. 5(b) is a schematic view showing the joined portions observed from laterally inside the joined portions (from the relatively close side to the center of the diaper in the lateral direction).
[FIGS. 6] FIG. 6(a) is a view of another embodiment of the absorbent article in the present invention corresponding to FIG. 2; and FIG. 6(b) is a cross-sectional view of the absorbent article shown in FIG. 6(a) in a natural state (corresponding to FIG. 4).
[FIGS. 7] FIG. 7(a) is a view of still another embodiment of the absorbent article in the present invention corresponding to FIG. 2; and FIG. 7(b) is a cross-sectional view of the absorbent article shown in FIG. 7(a) in a natural state (corresponding to FIG. 4).
[FIGS. 8] FIG. 8(a) is a view of still another embodiment of the absorbent article in the present invention corresponding to FIG. 2; and FIG. 8(b) is a cross-sectional view of the absorbent article shown in FIG. 8(a) in a natural state (corresponding to FIG. 4).

### Description of Embodiments

Conventional absorbent articles including leak-proof cuffs have room for improvement in leakage prevention performance of the leak-proof cuffs. The present invention relates to an absorbent article having excellent leakage prevention performance of leak-proof cuffs and capable of effectively preventing lateral leakage.

The present invention will now be described on the basis of preferred embodiments thereof with reference to drawings. In the following description of drawings, identical or similar components are indicated by an identical or similar sign. Drawings are basically schematic, and dimensional ratios and the like may differ from the actual ones.

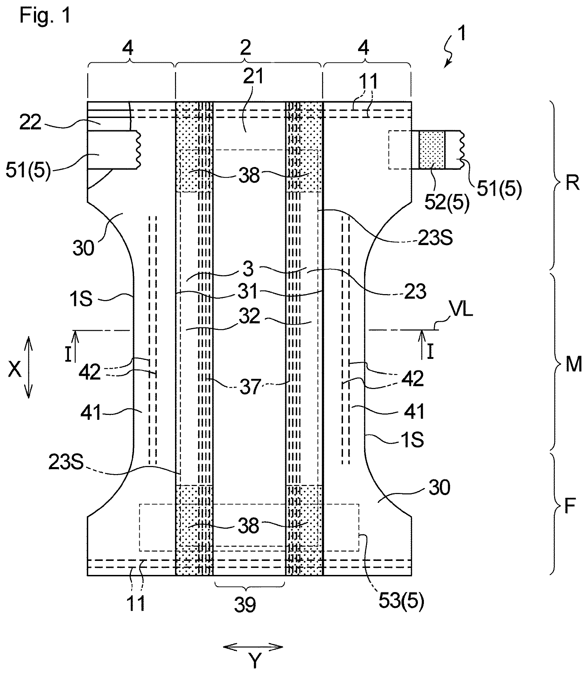

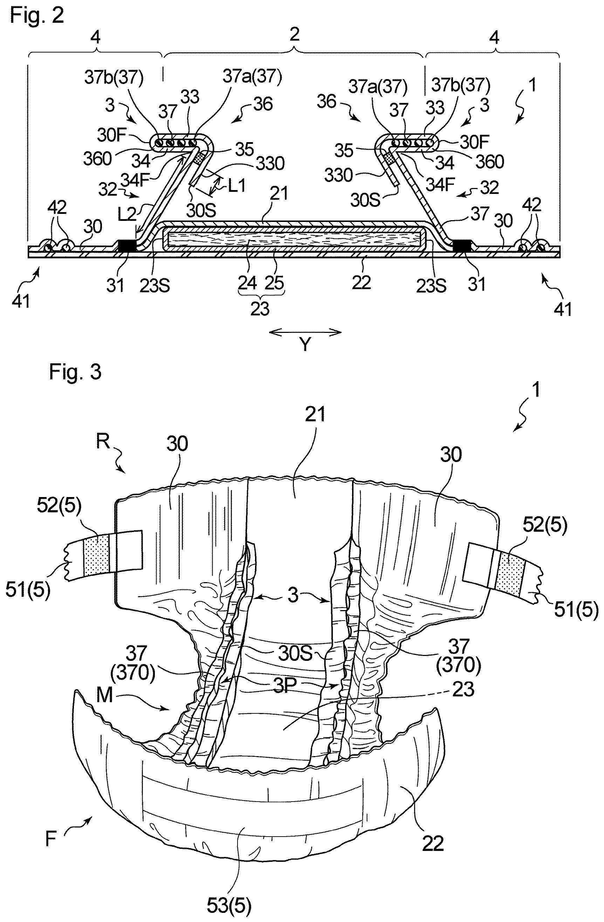

FIG. 1 and FIG. 2 show a disposable diaper 1 as an embodiment of the absorbent article in the present invention. The diaper 1 has a longitudinal direction X coincident with the front-rear direction of a wearer or with the direction extending from the front side through the crotch to the rear side and a lateral direction Y orthogonal thereto and includes an absorbent assembly 2 including an absorbent member 23 and a pair of leak-proof cuffs 3 and 3 extending in the longitudinal direction X and provided on a skin-facing surface of the absorbent assembly 2 while a separating portion 39 is interposed therebetween.

In the present description, a "skin-facing surface" is one surface of an absorbent article or a constituent member thereof (for example, an absorbent assembly) and is a face facing the skin of a wearer at the time of wearing the absorbent article or is the face relatively close to the skin of a wearer, and a "non-skin-facing surface" is the other surface of an absorbent article or a constituent member thereof and is a face facing opposite to the skin of a wearer at the time of wearing the absorbent article or is the face relatively distant from the skin of a wearer. In the present description, "at the time of wearing" means a condition in which a typical, appropriate wearing position or a normal wearing position of the absorbent article is maintained.

The diaper 1 (absorbent assembly 2) in the present embodiment, as shown in FIG. 1, includes a crotch portion M to be placed on the crotch of a wearer wearing the diaper, a front portion F to be placed on the anterior side to the crotch portion M in the longitudinal direction X or on the front side of the wearer, and a rear portion R to be placed on the posterior side to the crotch portion M in the longitudinal direction X or on the rear side of the wearer. The crotch portion M includes an excretory-facing portion (not shown) to be placed to face the excretory such as the penis of a wearer wearing the diaper.

The absorbent assembly 2 includes the absorbent member 23, a topsheet 21 placed on a skin-facing surface of the absorbent member 23, and a backsheet 22 placed on a non-skin-facing surface of the absorbent member 23, and these members are integrated by a known joining means such as an adhesive to form the absorbent assembly. The absorbent member 23 is interposed between the topsheet 21 and the backsheet 22 and includes a liquid-retentive absorbent core 24 containing a water absorbing material and a core-wrap sheet 25 overlying the outer surface (the skin-facing surface and the non-skin-facing surface) of the absorbent core 24. The absorbent assembly 2 in the present embodiment has a rectangular shape in plan view and extends in the longitudinal direction X from the front portion F to the rear portion R, and the length direction thereof is coincident with the longitudinal direction X.

As each of the topsheet 21, the backsheet 22, and the absorbent member 23, various materials conventionally used in such absorbent articles can be used without any limitation. As the topsheet 21, for example, a single layer or multilayer nonwoven fabric or porous film having liquid permeability can be used. As the backsheet 22, a waterproof sheet, that is, a sheet having liquid impermeability (no liquid passes therethrough) or having sparingly liquid permeability (liquid is not completely prevented from passing therethrough but is difficult to pass) can be used, and, for example, a moisture permeable resin film or a laminate of a resin film and a nonwoven fabric can be used. As the absorbent core 24 included in the absorbent member 23, for example, an fiber stack prepared by accumulating a water absorbing material such as wood pulp and a water absorbing polymer or a sheet-like absorbing structure containing the water absorbing material can be used. As the core-wrap sheet 25 included in the absorbent member 23, a liquid permeable sheet can be used, and, for example, paper or a nonwoven fabric can be used.

The diaper 1 in the present embodiment includes a pair of side flaps 4 and 4 including members extending outward in the lateral direction Y from both side edges 23S and 23S along the longitudinal direction X of the absorbent member 23. In the present embodiment, as shown in FIG. 2, the topsheet 21 covers the whole region on the skin-facing surface of the absorbent member 23, whereas the backsheet 22 covers the whole region on the non-skin-facing surface of the absorbent member 23, and both the sheets 21 and 22 further extend outward in the lateral direction Y from the side edges 23S and 23S of the absorbent member 23 to form the side flaps 4 together with the leak-proof cuff forming sheets 30 described later. As described above, the side flaps 4 in the present embodiment are located in regions without the absorbent member 23 and includes the topsheet 21, the backsheet 22, and the leak-proof cuff forming sheets 30. The plurality of members included in the side flaps 4 are joined together by a known joining means such as an adhesive, heat sealing, and ultrasonic sealing.

In a region included in each of the pair of side flaps 4, 4 and to be placed around the corresponding leg of a wearer, a leg cuff 41 is formed. The leg cuff 41 is a part of the side flap 4, includes at least the leak-proof cuff forming sheet 30 and leg-cuff forming elastic members 42 fixed in a stretched state to the sheet 30, and in the present embodiment, further includes the backsheet 22 as shown in FIG. 2. In the present embodiment, on each of the pair of leg cuffs 41 and 41 (side flaps 4 and 4), a plurality of (specifically two) filamentous elastic members 42 extending in the longitudinal direction X are arranged in the lateral direction Y, and each elastic member 42 extends in the longitudinal direction X at least over the entire length of the crotch portion M in the longitudinal direction X. The leg cuff 41 is typically prepared as follows: the elastic members 42 in a stretched state in the longitudinal direction X are joined to the sheets included in the leg cuff 41 (in the present embodiment, the leak-proof cuff forming sheet 30 and the backsheet 22) through a joining means such as an adhesive; and then the elastic members 42 are released from the stretched state. The leg cuffs 41 (side flaps 4) are contracted by a contraction force of the elastic members 42 and can be fitted around the legs of a wearer wearing the diaper 1.

In each end portion of the front portion F and the rear portion R in the longitudinal direction X, that is, in each waist end portion, waist gathering forming elastic members 11 are provided in a stretchable state in the lateral direction Y and extend in the lateral direction Y from one side flap 4 in the lateral direction Y to the other side flap 4. With such a structure, the waist end portions are contracted by a contraction force of the waist gathering forming elastic members 11 and can be fitted around the waist of a wearer wearing the diaper 1.

The diaper 1 is what is called an open type disposable diaper and includes an attachment structure 5 to be used at the time of wearing. The attachment structure 5, as shown in FIG. 1, includes a pair of fastening tapes 51 and 51 provided on the respective side edge portions along the longitudinal direction X in the rear portion R of the diaper 1 and a target region 53 provided on the non-skin-facing surface in the front portion F of the diaper 1 (the non-skin-facing surface of the backsheet 22). Each fastening tape 51 has an attachment portion 52, and the attachment portions 52 are attached to the target region 53 when the diaper 1 is worn. Onto the target region 53, the attachment portions 52 can be attached detachably. Typically, the attachment portion 52 includes a male member of a mechanical hook and loop fastener, whereas the target region 53 includes a female member of the mechanical hook and loop fastener.

The leak-proof cuffs 3, which are a main characteristic part of the diaper 1, will be described in detail. As shown in FIG. 1 and FIG. 2, each of the pair of leak-proof cuffs 3 and 3 includes a leak-proof cuff forming sheet 30 and has, in the lateral direction Y, a base portion 31 at which the sheet 30 is fixed to another member and a raising portion 32 that raises the sheet 30 toward a wearer from the base portion 31 as the starting point.

As shown in FIG. 1 and FIG. 2, a pair of the leak-proof cuff forming sheets 30 are provided in the respective side portions along the longitudinal direction X on the skin-facing surface of the absorbent assembly 2, and a separating portion 39 is interposed between the pair of leak-proof cuff forming sheets 30 and 30. The separating portion 39 is located at the center portion of the absorbent assembly 2 (diaper 1) in the lateral direction Y. Each leak-proof cuff forming sheet 30 is provided over the corresponding side edge 23S of the absorbent member 23 in the lateral direction Y and includes, in the lateral direction Y, a portion overlapping with the absorbent member 23 and a portion located outside the absorbent member 23 in the lateral direction Y in plan view.

As the leak-proof cuff forming sheet 30, a hydrophobic sheet material is used from the viewpoint of certainly achieving such a role as to prevent leakage of body fluids such as urine to the outside. As the hydrophobic sheet material, for example, a hydrophobic nonwoven fabric such as an air-through nonwoven fabric, a spunbonded nonwoven fabric, an SMS nonwoven fabric, an SMMS nonwoven fabric, and an SSMS nonwoven fabric can be used. In addition, a hydrophobic resin film, a laminate of the resin film and the nonwoven fabric, or the like can also be used. In the description, "S" means a spunbonded nonwoven fabric, and "M" means a meltblown nonwoven fabric.

The base portion 31 is a standing starting point when the raising portion 32 rises toward the skin of a wearer wearing the diaper 1. More specifically, the base portion 31 is in a fixing part of the leak-proof cuff forming sheet 30 to "another member" and is a portion adjacent to the raising portion 32 in the lateral direction Y, or is the innermost portion of the fixing part in the lateral direction Y. The "another member" is a constituent member of the diaper 1 except the leak-proof cuff forming sheet 30, is typically a member that is provided on the non-skin-facing surface of the sheet 30 and can be in contact with the sheet 30, and is, in the present embodiment, the topsheet 21 as shown in FIG. 2.

The base portion 31 is, as described above, a fixing part between the leak-proof cuff forming sheet 30 and another member and is typically formed by a known joining means such as an adhesive including hot melt and fusion bonding. The base portion 31 in the present embodiment, as shown in FIG. 1, has a straight-line shape in plan view, extends in the longitudinal direction X over substantially the entire length in the longitudinal direction X of the leak-proof cuff forming sheet 30, and is, as shown in FIG. 2, located outside the side edge 23S of the absorbent member 23 in the lateral direction Y and near the side edge 23S. The planar shape of the base portion 31 is not specifically limited and may be, for example, a straight-line shape, a wave shape, a curved shape, or a zigzag shape. The base portion 31 does not necessarily continue in one direction (longitudinal direction X) and may be a broken line in which fixing parts and non-fixing parts between the leak-proof cuff forming sheet 30 and another member are alternately arranged in the longitudinal direction X.

The raising portion 32 is a non-fixing part in the leak-proof cuff forming sheet 30 to another member. As shown in FIG. 2, the leak-proof cuff forming sheet 30 in the raising portion 32 is folded along a fold portion 30F extending in the longitudinal direction X. The raising portion 32 is sectioned into a first portion 33 from the fold portion 30F to a side edge 30S of the sheet 30 along the longitudinal direction X and a second portion 34 from the fold portion 30F to the base portion 31 and includes a sheet overlapping portion 36 in which the first portion 33 and the second portion 34 facing together are joined through a joined portion 35. The side edge 30S of the first portion 33 in the leak-proof cuff forming sheet 30 is closer to the center of the diaper 1 in the lateral direction Y than the other side edge of the sheet 30 along the longitudinal direction X (or is the inner side edge in the lateral direction Y). Of both side edge portions of the leak-proof cuff forming sheet 30 along the longitudinal direction X, the relatively distant side edge portion from the center of the diaper 1 in the lateral direction Y (the outer side edge in the lateral direction Y) constitutes a side edge portion 1S of the diaper 1 along the longitudinal direction X (see FIG. 1). The fold portion 30F has a straight-line shape extending in the longitudinal direction X in the diaper 1 in such a flat-out, uncontracted state as shown in FIG. 1. The sheet overlapping portion 36 has a two-layer structure including two leak-proof cuff forming sheets 30 facing together.

In the present invention, a "flat-out, uncontracted state" of a diaper 1 (absorbent article) is a state in which the diaper 1 is developed as shown in FIG. 1, and the developed diaper 1 is flattened out by stretching elastic members of each portion into design sizes (equal to the sizes of a flattened diaper where the effect of the elastic members is completely eliminated). In a diaper 1 in a flat-out, uncontracted state, typically, raising portions 32 do not rise but fall, but in FIG. 2 showing the same state, the raising portions 32 is allowed to rise for simple explanation.

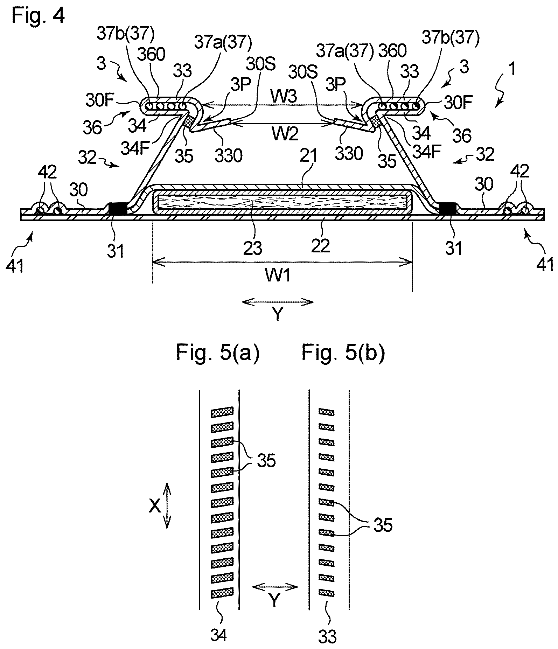

In the sheet overlapping portion 36, as shown in FIG. 2, the first portion 33 and the second portion 34 are joined at the joined portion 35 located closer to the fold portion 30F than the side edge 30S of the leak-proof cuff forming sheet 30. The joined portion 35 extends in the longitudinal direction X. In the description, "extending" includes not only a case in which a joined portion 35 continuously linearly extends in the longitudinal direction X but also, as shown in FIG. 5, a case in which a plurality of joined portions 35 are intermittently arranged in the longitudinal direction X and the plurality of joined portions 35 extend in the longitudinal direction X as a whole (the embodiment in FIG. 5 will be described later). At the joined portion 35, the first portion 33 and the second portion 34 may be joined through an adhesive or may be joined through fusion of a forming material such as fibers contained in the portions 33 and 34. The fusion between the first portion 33 and the second portion 34 may be achieved by a known fusing means such as heat sealing and ultrasonic sealing.

In the present embodiment, as shown in FIG. 2, between the first portion 33 and the second portion 34 included in the sheet overlapping portion 36, more specifically, between the fold portion 30F and the joined portion 35, a plurality of (four in the present embodiment) leak proof cuff forming elastic members 37 extending in the longitudinal direction X are arranged in the lateral direction Y in a stretchable state in the longitudinal direction X. In the sheet overlapping portion 36, a portion in which the leak proof cuff forming elastic members 37 are arranged in a stretchable state in the longitudinal direction X, or a portion between the fold portion 30F and the joined portion 35, is an elasticized portion 360 having elasticity in the longitudinal direction X. In other words, the sheet overlapping portion 36 has elasticity in the longitudinal direction X. The leak-proof cuff forming elastic member 37 is preferably filamentous or may be continuous instead. In the present embodiment, each sheet overlapping portion 36 has a plurality of leak proof cuff forming elastic members 37, but the number of the elastic members 37 is not specifically limited, and a single elastic member may be used.

In the present embodiment, as shown in FIG. 2, the second portion 34 (the portion from the fold portion 30F to the base portion 31 in the raising portion 32) is folded outward in the lateral direction Y along a fold portion 34F extending in the longitudinal direction X, and the sheet overlapping portion 36 extends outward in the lateral direction Y from the fold portion 34F. The second portion 34 folded outward as above allows the surface of the sheet overlapping portion 36, specifically, the surface of the first portion 33, to come into contact with the skin of a wearer wearing the diaper 1.

The leak-proof cuff 3 is typically prepared by the following method: leak-proof cuff forming elastic members 37 are fixed in a stretched state to a leak-proof cuff forming sheet 30; and then the stretched state is released. Due to the preparation method, many creases are formed on the surface of the sheet overlapping portion 36 that can come into contact with the skin of a wearer wearing the diaper 1, more specifically, on each surface of the first portion 33 and the second portion 34. The creases on the surface of the sheet overlapping portion 36 are intermittently arranged substantially regularly in the longitudinal direction X.

The raising portions 32 are preferably located in at least the crotch portion M. This is because the diaper 1 has a problem of lateral leakage particular in the crotch portion M in which excrements are mainly excreted. The raising portions 32 in the present embodiment, as shown in FIG. 1, continue over the entire length of the crotch portion M in the longitudinal direction X and further extend to both the front portion F and the rear portion R.

As shown in FIG. 1, in the respective outer regions of the raising portions 32 of the pair of leak-proof cuffs 3 and 3 in the longitudinal direction X, raising suppression portions 38 are formed. The raising portion 32 is interposed between a raising suppression portion 38 at one end in the longitudinal direction X (in the front portion F) and a raising suppression portion 38 at the other end (in the rear portion R). In the raising suppression portion 38, a portion coincident in the lateral direction Y with the raising portion 32 (i.e., the non-fixing part to another member) in the leak-proof cuff forming sheet 30 is fixed to another member (in the present embodiment, the topsheet 21) in the position overlapping with the absorbent member 23 in plan view. This prevents the raising suppression portion 38 from rising even when the raising portion 32 rises.

At the time of wearing the diaper 1, each raising portion 32 of the pair of leak-proof cuffs 3 and 3 rises due to a contraction force of the leak proof cuff forming elastic members 37 provided in the sheet overlapping portion 36, from the base portion 31 as a standing base. Accordingly, a pair of leak-proof walls are formed from the raising portions 32 at the respective sides along the longitudinal direction X, on the skin-facing surface of the absorbent assembly 2 in at least the crotch portion M. The pair of leak-proof walls dam excrements such as urine and feces excreted to the skin-facing surface of the diaper 1 and prevent the excrements from leaking outside the diaper 1 in the lateral direction Y, or prevent what is called lateral leakage.

At the time of wearing the diaper 1, the diaper 1 is curved by a contraction force of the leak-proof cuff forming elastic members 37 in such a way that the center portion of the diaper 1 in the longitudinal direction X (a portion including the crotch portion M), or a portion with the elastic members 37, forms a convex shape on the non-skin-facing surface (on the backsheet 22), or forms a concave shape on the skin-facing surface (on the topsheet 21). Accordingly, the diaper 1 is deformed into a boat shape as a whole. Such a boat-shaped diaper 1 easily fits with the crotch shape of a wearer. Hence, the sheet overlapping portions 36 of the raising portions 32 in a rising state in the diaper 1 come into close contact with the skin of a wearer with satisfactory fitting performance, and accordingly, the lateral leakage prevention function by the leak-proof cuffs 3 can be effectively achieved.

One of the main characteristics of the diaper 1 is that the first portion 33 of the raising portion 32, or the portion from the fold portion 30F to the side edge 30S of the leak-proof cuff forming sheet 30 in the raising portion 32, has a free edge portion 330 that is included in the sheet 30, extends from the joined portion 35, and is not fixed to any other member, as shown in FIG. 2. The free edge portion 330 of the first portion 33 extends in the longitudinal direction X over the entire length of the raising portion 32 in the longitudinal direction X.

The free edge portion 330 of the first portion 33 at least in such a diaper 1 in a natural state as shown in FIG. 3 recurves from the joined portion 35 as the starting point toward the elasticized portion 360 (the portion between the fold portion 30F and the joined portion 35 in the first portion 33) also as shown in FIG. 4, and a pocket structure 3P is formed between the recurved free edge portion 330 and the elasticized portion 360. The diaper 1 is almost in a natural state at the time of wearing, and thus the pocket structure 3P extends in the longitudinal direction X along the raising portion 32 in the rising state at the time of wearing the diaper 1.

In the present invention, a "natural state" of a diaper 1 (absorbent article) is a state (relaxation state) of the diaper 1 without any external force and is more specifically a state when each elasticized portion 360 of leak-proof cuffs 3 having elasticity in the longitudinal direction X (the portion with leak proof cuff forming elastic members 37 fixed in a stretched state) has an elongation of 0. In the description, "an elasticized portion 360 having an elongation of 0" means a state in which all leak proof cuff forming elastic members 37 in the elasticized portion 360 are not stretched, and the elasticized portion 360 has a minimum length in the longitudinal direction X.

In the present embodiment, as shown in FIG. 2 and FIG. 4, the first portion 33 is located inside the second portion 34 in the lateral direction Y. In other words, the first portion 33 is relatively close to the center of the diaper 1 in the lateral direction Y, whereas the second portion 34 is relatively distant from the center of the diaper 1 in the lateral direction Y. When the positional relation between such a first portion 33 and a second portion 34 in the lateral direction Y is relatively determined, and the second portion 34 is folded as in the present embodiment, the folding is released to spread the second portion 34, and then the positional relation is determined.

When the first portion 33 is located inside the second portion 34 in the lateral direction Y, the pocket structure 3P formed by the recurved free edge portion 330 of the first portion 33 is located inside the leak-proof cuff 3 (raising portion 32) in the lateral direction Y as shown in FIG. 4 and thus is located in a region between the pair of leak-proof cuffs 3 and 3. Such a pocket structure 3P formed inside the leak-proof cuff 3 becomes a barrier to excrements that would climb over the leak-proof cuff 3 and can prevent excrements from inexpediently climbing over the leak-proof cuff 3. In other words, by adopting the structure in which "the free edge portion 330 of the first portion 33 is not fixed to any other member but extends from the joined portion 35 between the first portion 33 and the second portion 34 included in the sheet overlapping portion 36", the pocket structure 3P that can prevent excrements from climbing over the leak-proof cuff 3 to go outside in the lateral direction Y is added to the leak-proof cuff 3. This improves the leakage prevention performance of the leak-proof cuff 3 and can effectively prevent lateral leakage.

The extension length L1 of the free edge portion 330 of the first portion 33 from the joined portion 35 (see FIG. 2) is intended to be not less than 0.05 times the length L2 from the joined portion 35 to the base portion 31 in the second portion 34 (see FIG. 2). Each length L1 and L2 is a length when a sheet included in the corresponding portion (leak-proof cuff forming sheet 30) is stretched and spread to design sizes (equal to sizes when the sheet is flattened out into a plane while the effects of elastic members are completely eliminated). If L1/L2 is less than 0.05, a free edge portion 330 is excessively short relative to the length L2 relevant to the rising height of a raising portion 32. Hence, advantageous effects by a free edge portion 330, specifically, for example, the above advantageous effect by a pocket structure 3P is insufficiently achieved, and leakage prevention performance of a leak-proof cuff 3 is insufficiently improved.

From the viewpoint of more certainly achieving the advantageous effect by the free edge portion 330 of the first portion 33, L1/L2 is preferably 0.1 or more, more preferably 0.15 or more, and even more preferably 0.2 or more. The upper limit of L1/L2 is not specifically limited. From the viewpoint of allowing the free edge portion 330 to function as the pocket structure, L1/L2 is preferably 0.9 or less, more preferably 0.8 or less, and even more preferably 0.7 or less.

The extension length L1 of the free edge portion 330 of the first portion 33 from the joined portion 35 (see FIG. 2) is preferably 2 mm or more and more preferably 4 mm or more and is preferably 25 mm or less and more preferably 20 mm or less.

The length L2 from the joined portion 35 to the base portion 31 in the second portion 34 is preferably 5 mm or more and more preferably 10 mm or more and is preferably 30 mm or less and more preferably 25 mm or less.

As shown in FIG. 4, when the first portion 33 is located inside the second portion 34 in the lateral direction Y, W2 < W1 and W2 < W3 are preferably satisfied in the diaper 1 in a natural state as shown in FIG. 3, where W1 is the distance (minimum distance) between the base portion 31 of one leak-proof cuff 3 and that of the other leak-proof cuff of the pair of leak-proof cuffs 3 and 3, W2 is the distance (minimum distance) between the free edge portion 330 of one leak-proof cuff forming sheet 30 and that of the other leak-proof cuff forming sheet, and W3 is the distance (minimum distance) between one sheet overlapping portion 36 and the other sheet overlapping portion. When the above magnitude relations are satisfied, the pocket structure 3P can satisfactory function as a barrier to excrements that would climb over the leak-proof cuff 3 at the time of wearing the diaper 1.

When the first portion 33 is located inside the second portion 34 in the lateral direction Y, dimensions and the like of portions are preferably set to the following values.

The ratio of the distance W1 to the distance W2, W1/W2, is preferably 1.01 or more and more preferably 1.05 or more and is preferably 2.0 or less and more preferably 1.5 or less, provided that W1 > W2.

The ratio of the distance W3 to the distance W2, W3/W2, is preferably 1.01 or more and more preferably 1.05 or more and is preferably 1.9 or less and more preferably 1.4 or less, provided that W3 > W2.

The distance W1 is preferably 115 mm or more and more preferably 120 mm or more and is preferably 145 mm or less and more preferably 140 mm or less.

The distance W2 is preferably 75 mm or more and more preferably 80 mm or more and is preferably 135 mm or less and more preferably 130 mm or less.

The distance W3 is preferably 100 mm or more and more preferably 105 mm or more and is preferably 140 mm or less and more preferably 135 mm or less.

According to the invention, as shown in FIG. 2 and the like, a plurality of (in the present embodiment, four) leak proof cuff forming elastic members 37 extending in the longitudinal direction X are arranged in the lateral direction Y in a stretchable state in the longitudinal direction X between the first portion 33 and the second portion 34. At the time of wearing the diaper 1, the innermost elastic member in the lateral direction Y (indicated by sign 37a in the drawing) of the plurality of elastic members 37 preferably has a larger stress than the outermost elastic member in the lateral direction Y (indicated by sign 37b in the drawing). In this manner, at the time of wearing the diaper 1, when the magnitude relation of "the stress of the laterally innermost elastic member 37a > the stress of the laterally outermost elastic member 37b" is satisfied, the free edge portion 330 of the first portion 33 extending from the j oined portion 35 easily recurves, and the pocket structure 3P is easily formed.

The above "time of wearing a diaper 1 (absorbent article)" is a condition in which the "cuff longitudinal length" is 30 or more and 90 or less. In the description, a "cuff longitudinal length" is a relative longitudinal length where a leak-proof cuff included in an absorbent article (the leak-proof cuff 3 in the diaper 1) in a fully stretched state has a longitudinal length of 100. The cuff longitudinal length is typically minimum when a leak-proof cuff is in a natural state (no external force is applied), but the minimum length is not zero.

At the time of wearing the diaper 1, the ratio of the stress of the laterally innermost elastic member 37a to the stress of the laterally outermost elastic member 37b, the former/the latter, is preferably 1.01 or more and more preferably 1.05 or more and is preferably 3 or less and more preferably 2 or less, provided that the former > the latter.

At the time of wearing the diaper 1, the stress of the laterally innermost elastic member 37a is preferably 0.03 N or more and more preferably 0.06 N or more and is preferably 0.24 N or less and more preferably 0.2 N or less.

At the time of wearing the diaper 1, the stress of the laterally outermost elastic member 37b is preferably 0.02 N or more and more preferably 0.05 N or more and is preferably 0.2 N or less and more preferably 0.16 N or less.

The stress of an elastic member including leak proof cuff forming elastic members 37 is determined by the following method.

### <Measurement method of stress>

From a sheet overlapping portion 36 (elasticized portion 360), a leak proof cuff forming elastic member to be measured (the innermost elastic member 37a in the lateral direction Y, the outermost elastic member 37b in the lateral direction Y) is cut out as a measurement sample. The measurement sample has a longitudinal length (length in the longitudinal direction X) of 100 mm. The respective longitudinal ends of a measurement sample are clamped to chucks of a Tensilon universal tester (RTC-1210A) manufactured by ORIENTEC. The measurement sample is then stretched in the length direction (longitudinal direction X) to a fully stretched state by expanding the chuck distance at a speed of 300 mm/min, and the measurement sample is contracted to a predetermined length by reducing the chuck distance at a speed of 300 mm/min. The force (contractive force) (unit: N) during the contracting is determined. To determine the stress of a measurement sample (leak proof cuff forming elastic member 37a or 37b) at the time of wearing the diaper 1, the stress is, as apparent from the above definition "time of wearing a diaper 1 (absorbent article)", a contraction stress at a cuff longitudinal length of 30 or more and 90 or less where a measurement sample in a fully stretched state has a cuff longitudinal length of 100, and thus, in the present measurement, a measurement sample in a fully stretched state corresponding to a cuff longitudinal length of 100 is contracted to a length corresponding to a cuff longitudinal length of 30.

The method for satisfying the magnitude relation "the stress of the laterally innermost elastic member 37a > the stress of the laterally outermost elastic member 37b" at the time of wearing the diaper 1, or the method for making the free edge portion 330 of the first portion 33 extending from the joined portion 35 easily recurve to facilitate the formation of the pocket structure 3P, for example, include a method of making the laterally innermost elastic member 37a have a larger thickness than the laterally outermost elastic member 37b.

Making the free edge portion 330 of the first portion 33 extending from the joined portion 35 easily recurve can also be achieved by improving the joined portion 35. Specifically, when the joined portion 35 is a fused portion at which the first portion 33 is fused with the second portion 34, by making the area of the joined portion 35 (fused portion) observed from the first portion 33 (hereinafter also called "fused area on the first portion") different from the area of the joined portion 35 observed from the second portion 34 (hereinafter also called, "fused area on the second portion"), the free edge portion 330 is facilitated to recurve , and the pocket structure 3P can be easily formed.

FIGS. 5 show an embodiment in which the fused area on the first portion differs from the fused area on the second portion. FIGS. 5 are each a view of one set of joined portions 35 (fused portions) of a pair of leak-proof cuffs 3 and 3 observed in the extending direction (longitudinal direction X) in a diaper 1; FIG. 5(a) is a view of the joined portions 35 observed from outside in lateral direction Y (from the relatively distant side from the center of the diaper 1 in the lateral direction Y); and FIG. 5(b) is a view of the joined portions 35 observed from inside in the lateral direction Y (from the relatively close side to the center of the diaper 1 in the lateral direction Y). In the diaper 1, as described above, the first portion 33 is located inside the second portion 34 in the lateral direction Y, and thus FIG. 5(a) is a view of the joined portions 35 observed along the longitudinal direction X from the second portion 34 (from outside in the lateral direction Y), whereas FIG. 5(b) is a view of the joined portions 35 observed along the longitudinal direction X from the first portion 33 (from inside in the lateral direction Y). In the embodiment shown in FIGS. 5, in each of the observation from the first portion 33 and the observation from the second portion 34, a plurality of joined portions 35 (fused portions) are intermittently arranged in the longitudinal direction X. The area of each joined portion 35 (fused area) is larger in the observation from the second portion 34 than in the observation from the first portion 33, and in a unit region of the leak-proof cuff 3 (leak-proof cuff forming sheet 30), specifically, for example, in a quadrangular region in plan view having a length of 10 mm in the longitudinal direction X and a length of 10 mm in the lateral direction Y (a region corresponding to a "measurement sheet" in the fused area measurement method described later ), the magnitude relation "the fused area on the first portion < the fused area on the second portion" is satisfied. The fused area (the area of a joined portion) is determined by the following method.

### <Measurement method of fused area>

A leak-proof cuff 3 (leak-proof cuff forming sheet 30) is cut out from a diaper 1, and from the cut-out leak-proof cuff 3, a 10 mm × 10 mm quadrangular sheet in plan view including joined portions 35 (fused portions) is cut out as a measurement sheet. Both faces of the measurement sheet (the face on the first portion 33 and the face on the second portion 34) are analyzed by using an image analyzing device, for example, VHX-1000 manufactured by KEYENCE, to determine a fused area from the outline of a joined portion 35. In the fused area measurement, the areas of all of one or more joined portions 35 (excluding cut-out or incomplete joined portions 35) on a measurement face of the measurement sheet (on the first portion 33 or on the second portion 34) are measured to give the fused area on the measurement face. For a measurement face having a plurality of joined portions 35, the average area of the plurality of joined portions 35 is calculated as the fused area of the measurement face.

When a first portion 33 is located inside a second portion 34 in the lateral direction Y as in the diaper 1 and the diaper 1A described later, satisfying a magnitude relation "the fused area on a first portion < the fused area on a second portion" as shown in FIG. 5 can facilitate recurving a free edge portion 330 of the first portion 33. Meanwhile, when a first portion 33 is located outside a second portion 34 in the lateral direction Y as in the diapers 1B and 1C described later, satisfying a magnitude relation "the fused area on a first portion > the fused area on a second portion" on the contrary to the embodiment shown in FIG. 5 can facilitate recurving a free edge portion 330 of the first portion 33.

Making the fused area on a first portion different from the fused area on a second portion can be achieved by fusing a sheet at positions planned to form joined portions 35 (fused portions) from both the first portion 33 and the second portion 34 or can be achieved by fusing a sheet from only one of the first portion 33 and the second portion 34. The fusing can be performed by a known fusing means such as heat sealing and ultrasonic sealing. When fusing is performed from only one of the first portion 33 and the second portion 34 to make the fused area on the first portion different from the fused area on the second portion, a fusing means using ultrasonic waves, such as ultrasonic sealing, is preferably used as the fusing means.

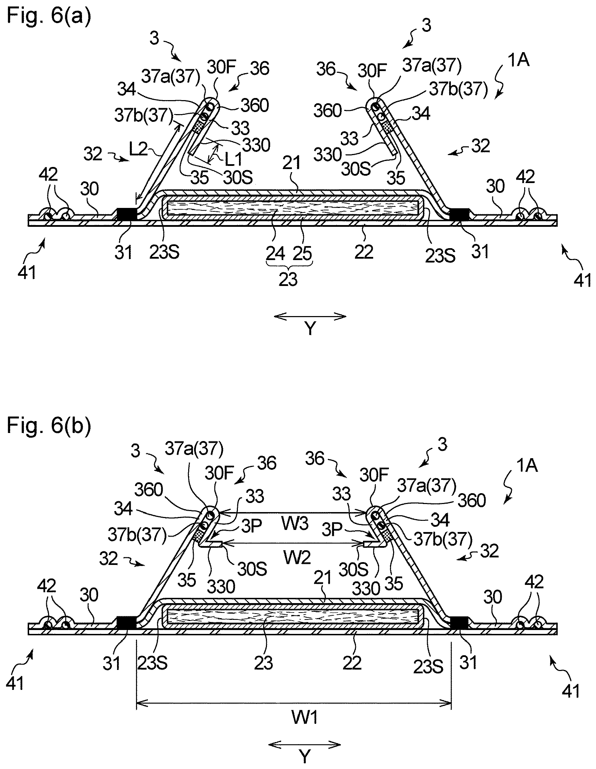

FIG. 6 to FIG. 8 show other embodiments of the absorbent article in the present invention. The embodiments described later will mainly describe components different from the above diaper 1, and a similar component is indicated by the same sign and is not described. To components not specifically described, the description of the diaper 1 will be appropriately applied.

In the diaper 1, the second portion 34 of the raising portion 32 is folded outward in the lateral direction Y (see FIG. 2 and FIG. 4). In a diaper 1A shown in FIG. 6, a second portion 34 is not folded, but a raising portion 32 entirely extends from a base portion 31 in one direction (direction from outside to inside in the lateral direction Y). In the diaper 1A, two leak proof cuff forming elastic members 37 are arranged in each of a pair of sheet overlapping portions 36 and 36, and the number of the elastic members 37 in each sheet overlapping portion 36 is smaller than in the diaper 1. The diaper 1A has the same structure as the diaper 1 except the above structure and satisfies W2 < W1 and W2 < W3 in a natural state. The diaper 1A also basically exerts substantially the same effect as the diaper 1, but from the viewpoint of certainly preventing excrements from inexpediently climbing over leak-proof cuffs 3, the second portions 34 are preferably folded outward in the lateral direction Y on fold portions 34F as in the diaper 1.

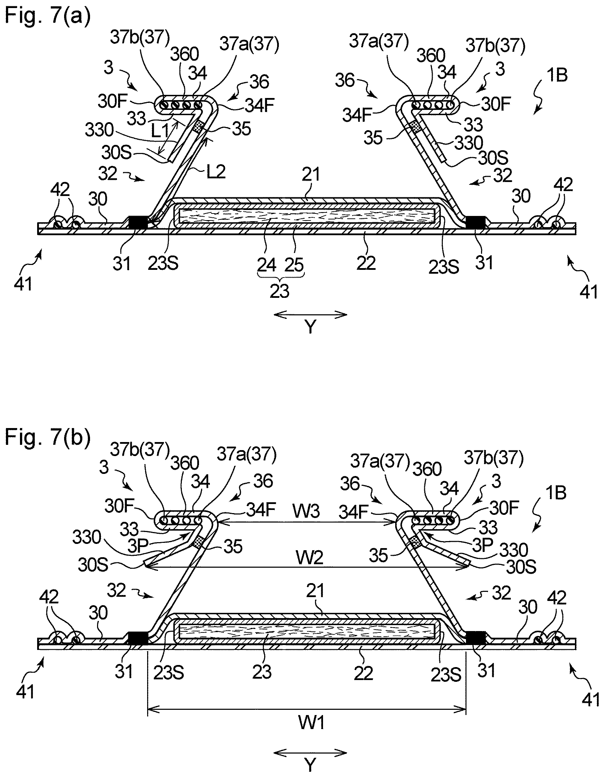

In a diaper 1B shown in FIG. 7, a first portion 33 is located outside a second portion 34 in the lateral direction Y, and the arrangement of both portions 34 and 34 is opposite to those of the diapers 1, 1A. In other words, in the diaper 1B, the first portion 33 is relatively distant from the center of the diaper 1B in the lateral direction Y, whereas the second portion 34 is relatively close to the center of the diaper 1B in the lateral direction Y. Due to this structure, a pocket structure 3P formed by a recurved free edge portion 330 of the first portion 33 is located outside a leak-proof cuff 3 (raising portion 32) in the lateral direction Y as shown in FIG. 7(b) and is accordingly located outside a region between the pair of leak-proof cuffs 3 and 3 in the lateral direction Y. Such a pocket structure 3P formed outside the leak-proof cuff 3 can catch excrements having climbed over the leak-proof cuff 3 to prevent the excrements from going outside the pocket structure 3P in the lateral direction Y and can prevent lateral leakage. In other words, by adopting the structure in which "a free edge portion 330 of a first portion 33 is not fixed to any other member but extends from a joined portion 35 between the first portion 33 and a second portion 34 included in a sheet overlapping portion 36", the pocket structure 3P that can catch excrements having climbed over the leak-proof cuff 3 is added to the leak-proof cuff 3. This improves the leakage prevention performance of the leak-proof cuff 3 and can effectively prevent lateral leakage.

As shown in FIGS. 7, when a first portion 33 is located outside a second portion 34 in the lateral direction Y, W2 > W1 and W2 > W3 are preferably satisfied in a diaper 1 in a natural state as shown in FIG. 3, where W1 is the distance between a base portion 31 of one leak-proof cuff 3 and that of the other leak-proof cuff of a pair of leak-proof cuffs 3 and 3, W2 is the distance between a free edge portion 330 of one leak-proof cuff forming sheet 30 and that of the other leak-proof cuff forming sheet, and W3 is the distance (minimum distance) between one sheet overlapping portion 36 and the other sheet overlapping portion. When the above magnitude relations are satisfied, the pocket structure 3P can satisfactory function as a pocket to catch excrements having climbed over the leak-proof cuff 3 at the time of wearing the diaper 1.

When a first portion 33 is located outside a second portion 34 in the lateral direction Y, dimensions and the like of portions are preferably set to the following values.

The ratio of the distance W1 to the distance W2, W1/W2, is preferably 0.6 or more and more preferably 0.65 or more and is preferably 0.99 or less and more preferably 0.95 or less, provided that W1 < W2.

The ratio of the distance W3 to the distance W2, W3/W2, preferably 0.55 or more and more preferably 0.6 or more and is preferably 0.95 or less and more preferably 0.9 or less, provided that W3 < W2.

The distance W1 is preferably 115 mm or more and more preferably 120 mm or more and is preferably 145 mm or less and more preferably 140 mm or less.

The distance W2 is preferably 120 mm or more and more preferably 125 mm or more and is preferably 180 mm or less and more preferably 175 mm or less.

The distance W3 is preferably 100 mm or more and more preferably 105 mm or more and is preferably 140 mm or less and more preferably 135 mm or less.

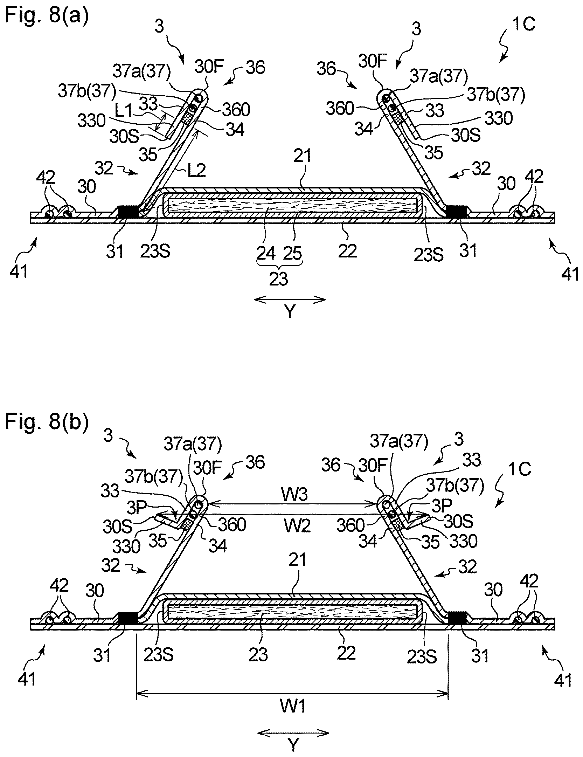

In the diaper 1B, the second portion 34 of the raising portion 32 is folded outward in the lateral direction Y on a fold portion 34F (see FIG. 7). In a diaper 1C shown in FIG. 8, a second portion 34 is not folded, but a raising portion 32 entirely extends from a base portion 31 in one direction (direction from outside to inside in the lateral direction Y). In the diaper 1C, two leak proof cuff forming elastic members 37 are arranged in each of a pair of sheet overlapping portions 36 and 36, and the number of the elastic members 37 in each sheet overlapping portion 36 is smaller than in the diaper 1B. The diaper 1C has the same structure as the diaper 1B except the above structure and satisfies W2 > W1 and W2 > W3 in a natural state. The diaper 1C also exerts substantially the same effect as the diaper 1B.

The present invention has been described hereinabove, but the present invention is not limited to the above embodiments and can be appropriately modified without departing from the scope of the present invention.

The absorbent article of the present invention widely encompasses articles used for absorption of body fluids excreted from a human body (such as urine, menstrual blood, loose feces, and sweat) and can encompass, in addition to such open type disposable diapers as in the above embodiments, pull-on disposable diapers, incontinence pads, sanitary napkins, sanitary shorts, and the like.

### Examples

The present invention will next be described more specifically with reference to examples, but the present invention is not limited to the examples.

### [Examples 1 and 2, Comparative Example 1]

Open type disposable diapers having substantially the same basic configuration as that of the diaper 1 shown in FIG. 1, or absorbent articles in which in each raising portion of leak-proof cuffs, a first portion was located inside a second portion in the lateral direction, were prepared. Specifically, as an open type disposable diaper, Merries Tape S size manufactured by Kao Corporation (manufactured in 2017, registered trademark) was prepared, and dimensions and the like of components of the leak-proof cuffs of the prepared diaper were appropriately set, giving open type disposable diapers of examples and comparative examples.

### [Examples 3 and 4, Comparative Example 2]

Open type disposable diapers having substantially the same basic configuration as that of the diaper 1B shown in FIG. 7, or absorbent articles in which in each raising portion of leak-proof cuffs, a first portion was located outside a second portion in the lateral direction, were prepared. Specifically, as an open type disposable diaper, Merries Tape S size manufactured by Kao Corporation (manufactured in 2017, registered trademark) was prepared, and dimensions and the like of components of the leak-proof cuffs of the prepared diaper were appropriately set, giving open type disposable diapers of examples and comparative examples.

Lateral leakage prevention performance of the leak-proof cuffs of each disposable diaper of examples and comparative examples was evaluated by the following methods. The results are shown in Table 1.

### <Evaluation method of lateral leakage prevention performance of leak-proof cuffs>

A diaper to be evaluated was put on a human body model having an excretion point, and the lateral leakage prevention performance of leak-proof cuffs of the diaper was evaluated by the following method. First, the diaper was put on the human body model, and the human body model was tilted at 90° to face sideways. In this condition, 10 g of pseudo loose feces were discharged to a portion of the diaper corresponding to the excretion point. The pseudo loose feces were composed of 22.5% by mass of bentonite, 0.5% by mass of a surfactant (Poise 530, a solid content of 40% by mass, manufactured by Kao Corporation), 1.5% by mass of a 0.03% by mass aqueous solution of Emulgen 130K (manufactured by Kao Corporation), and 75.5% by mass of ion-exchanged water and had a viscosity of 40 mPa·s (23°C, vibration viscometer: SV-10 manufactured by A&D) and a surface tension of 55 mN/m. Immediately after discharge of the pseudo loose feces, the human body model was allowed to stand upright, and the diaper located on the crotch of the human body model was pressed toward the human body model at a pressure of 3 kPa. Whether or not the pseudo loose feces leaked outward in the lateral direction from the leak-proof cuffs of the diaper was observed. The above operation was performed 10 times for a single evaluation object, and the probability of successful prevention of lateral leakage (the proportion of the number of tests where no leakage of the pseudo loose feces was observed, to the 10 tests) was regarded as the lateral leakage prevention performance. A sample having a larger numerical value has higher lateral leakage prevention performance of leak-proof cuffs and has higher evaluation.

**[Table 1]**

| | | Example | | | | Comparative Example | |
|---|---|---|---|---|---|---|---|
| | | 1 | 2 | 3 | 4 | 1 | 2 |
| Raising portion of leak-proof cuff | Positional relation of first portion to second portion in lateral direction | Laterally inside second portion | Laterally inside second portion | Laterally outside second portion | Laterally outside second portion | Laterally inside second portion | Laterally outside second portion |
| | Extension length of free edge portion of first portion, L1 (mm) | 8 | 20 | 7 | 10 | 1 | 2 |
| | Length from joined portion to base portion in second portion, L2 (mm) | 17 | 22 | 22 | 12 | 29 | 45 |
| | L1/L2 | 0.47 | 0.91 | 0.32 | 0.83 | 0.03 | 0.04 |
| Evaluation of diaper | Lateral leakage prevention performance of leak-proof cuff | 100% | 70% | 90% | 80% | 30% | 40% |

### Industrial Applicability

The present invention provides an absorbent article having excellent leakage prevention performance of leak-proof cuffs and capable of effectively preventing lateral leakage.

## Claims

1. An absorbent article having a longitudinal direction (X) corresponding to a front-rear direction of a wearer and a lateral direction (Y) orthogonal to the longitudinal direction (X), the absorbent article comprising:
an absorbent assembly (2) including an absorbent member (23); and
a pair of leak-proof cuffs (3) extending in the longitudinal direction (X) and provided on a skin-facing surface of the absorbent assembly (2) while a separating portion (39) is interposed therebetween, wherein
each of the pair of leak-proof cuffs (3) includes a leak-proof cuff forming sheet (30) and has a base portion (31) at which the leak-proof cuff forming sheet (30) is fixed to another member and a raising portion (32) that raises the leak-proof cuff forming sheet (30) toward the wearer from the base portion (31) as a starting point,
the leak-proof cuff forming sheet (30) constituting the raising portion (32) is folded along a fold portion (30F) extending in the longitudinal direction (X),
the raising portion (32) is sectioned into a first portion (33) from the fold portion (30F) to a side edge of the leak-proof cuff forming sheet (30) along the longitudinal direction (X) and a second portion (34) from the fold portion (30F) to the base portion (31) and includes a sheet overlapping portion (36) in which the first portion (33) and the second portion (34) facing together are joined through a joined portion(35),
the first portion (33) includes a free edge portion (330), the free edge portion (330) is not fixed to any other member, and is an extension portion of the leak-proof cuff forming sheet (30) extending from the joined portion (35), and
an extension length L1 of the free edge portion (330) from the joined portion (35) is not less than 0.05 times a length L2 from the joined portion (35) to the base portion (31) in the second portion (34), **characterized in that**
between the first portion (33) and the second portion (34), a plurality of leak proof cuff forming elastic members (37) are arranged in the lateral direction (Y), and
an innermost elastic member (37a) located innermost in the lateral direction (Y) of the plurality of leak proof cuff forming elastic members (37) has a larger stress than an outermost elastic member (37b) located outermost in the lateral direction (Y) when the absorbent article is worn.

2. The absorbent article according to claim 1, wherein the extension length L1 of the free edge portion (330) from the joined portion (35) is not less than 0.1 times and not more than 0.9 times the length L2 from the joined portion (35) to the base portion (31) in the second portion (34).

3. The absorbent article according to claim 1 or 2, wherein the first portion (33) is located inside the second portion (34) in the lateral direction (Y).

4. The absorbent article according to claim 3, wherein the absorbent article satisfies W2 < W1 and W2 < W3 in a natural state, where W1 is a distance between the base portion (31) of one leak-proof cuff and the base portion (31) of the other leak-proof cuff of the pair of leak-proof cuffs (3), W2 is a distance between the free edge portion (330) of the leak-proof cuff forming sheet (30) of one leak-proof cuff and the free edge portion (330) of the leak-proof cuff forming sheet (30) of the other leak-proof cuff, and W3 is a distance between the sheet overlapping portion (36) of one leak-proof cuff and the sheet overlapping portion (36) of the other leak-proof cuff.

5. The absorbent article according to claim 4, wherein a ratio of W1 to W2 (W1/W2) is 1.01 or more and 2.0 or less.

6. The absorbent article according to claim 4 or 5, wherein a ratio of W3 to W2 (W3/W2) is 1.01 or more and 1.9 or less.

7. The absorbent article according to claim 1 or 2, wherein the first portion (33) is located outside the second portion (34) in the lateral direction (Y).

8. The absorbent article according to claim 7, wherein the absorbent article satisfies W2 > W1 and W2 > W3 in a natural state, where W1 is a distance between the base portion (31) of one leak-proof cuff and the base portion (31) of the other leak-proof cuff of the pair of leak-proof cuffs (3) , W2 is a distance between the free edge portion (330) of the leak-proof cuff forming sheet (30) of one leak-proof cuff and the free edge portion (330) of the leak-proof cuff forming sheet (30) of the other leak-proof cuff, and W3 is a distance between the sheet overlapping portion (36) of one leak-proof cuff and the sheet overlapping portion (36) of the other leak-proof cuff.

9. The absorbent article according to claim 8, wherein a ratio of W1 to W2 (W1/W2) is 0.6 or more and 0.99 or less.

10. The absorbent article according to claim 8 or 9, wherein a ratio of W3 to W2 (W3/W2) is 0.55 or more and 0.95 or less.

11. The absorbent article according to an one of claims to 10, wherein a ratio of the stress of the innermost elastic member (37a) to the stress of the outermost elastic member (37b) is 1.01 or more and 3 or less.

12. The absorbent article according to any one of claims 1 to 11, wherein the stress of the innermost elastic member (37b) is 0.03 N or more and 0.24 N or less.

13. The absorbent article according to any one of claims 1 to 12, wherein the stress of the outermost elastic member (37b) is 0.02 N or more and 0.2 N or less.

14. The absorbent article according to any one of claims 1 to 13, wherein the joined portion (35) is a fused portion at which the first portion (33) is fused with the second portion (34), and an area of the fused portion observed from the first portion (33) differs from an area of the fused portion observed from the second portion (34).

## Patentansprüche

1. Absorbierender Artikel mit einer Längsrichtung (X), die einer Vorn-Hinten-Richtung eines Trägers entspricht, und einer Querrichtung (Y), die orthogonal zu der Längsrichtung (X) ist, wobei der absorbierende Artikel aufweist:
eine absorbierende Anordnung (2) mit einem absorbierenden Element (23); und
ein Paar austrittssichere Bündchen (3), die sich in der Längsrichtung (X) erstrecken und auf einer der Haut zugewandten Oberfläche der absorbierenden Anordnung (2) vorgesehen sind, während ein Trennabschnitt (39) zwischen ihnen angeordnet ist, wobei
jeder des Paars von austrittssicheren Bündchen (3) eine austrittssichere Bündchen-Materialbahn (30) beinhaltet und einen Basisabschnitt (31) aufweist, an dem die austrittssichere Bündchen-Materialbahn (30) an einem anderen Element befestigt ist, und einen Anhebeabschnitt (32) aufweist, der die austrittssichere Bündchen-Materialbahn (30) hin zu dem Träger von dem Basisabschnitt (31) als Ausgangspunkt anhebt,
die austrittssichere Bündchen-Materialbahn (30), die den Anhebeabschnitt (32) bildet, entlang eines sich in der Längsrichtung (X) erstreckenden Faltabschnitts (30F) gefaltet ist,
der Anhebeabschnitt (32) in einen ersten Abschnitt (33) von der Faltlinie (30F) bis zu einer Seitenkante der austrittssicheren Bündchen-Materialbahn (30) entlang der Längsrichtung (X) und einen zweiten Abschnitt (34) von dem Faltabschnitt (30F) bis zu dem Basisabschnitt (31) unterteilt ist und einen die Materialbahn überlappenden Abschnitt (36) aufweist, in dem der erste Abschnitt (33) und der zweite Abschnitt (34), die einander gegenüberliegen, über einen Verbindungsabschnitt (35) verbunden sind,
der erste Abschnitt (33) einen freien Randabschnitt (330) aufweist, der freie Randabschnitt (330) an keinem anderen Element befestigt ist und ein Verlängerungsabschnitt der austrittssicheren Bündchen-Materialbahn (30) ist und sich von dem Verbindungsabschnitt (35) aus erstreckt, und
eine Erstreckungslänge L1 des freien Randabschnitts (330) von dem Verbindungsabschnitt (35) nicht weniger als das 0,05-fache einer Länge L2 von dem Verbindungsabschnitt (35) bis zu dem Basisabschnitt (31) in dem zweiten Abschnitt (34) beträgt, **dadurch gekennzeichnet, dass**
zwischen dem ersten Abschnitt (33) und dem zweiten Abschnitt (34) mehrere elastische, austrittssichere Bündchenelemente (37) in seitlicher Richtung (Y) angeordnet sind, und
ein innerstes elastisches Element (37a), das in der seitlichen Richtung (Y) der mehreren elastischen austrittssicheren Bündchenelemente (37) am innersten liegt, eine höhere Spannung aufweist als ein äußerstes elastisches Element (37b), das in der seitlichen Richtung (Y) am weitesten außen liegt, wenn der absorbierende Artikel getragen wird.

2. Absorbierender Artikel nach Anspruch 1, wobei die Erstreckungslänge L1 des freien Randabschnitts (330) von dem Verbindungsabschnitt (35) nicht weniger als das 0,1-fache und nicht mehr als das 0,9-fache der Länge L2 von dem Verbindungsabschnitt (35) bis zu dem Basisabschnitt (31) in dem zweiten Abschnitt (34) beträgt.

3. Absorbierender Artikel nach Anspruch 1 oder 2, wobei sich der erste Abschnitt (33) in der seitlichen Richtung (Y) innerhalb des zweiten Abschnitts (34) befindet.

4. Absorbierender Artikel nach Anspruch 3, wobei der absorbierende Artikel in einem natürlichen Zustand W2 < W1 und W2 < W3 erfüllt, wobei W1 ein Abstand zwischen dem Basisabschnitt (31) eines austrittssicheren Bündchens und dem Basisabschnitt (31) des anderen austrittssicheren Bündchens des Paars austrittssicherer Bündchen (3) ist, W2 ein Abstand zwischen dem freien Randabschnitt (330) der austrittssicheren Bündchen-Materialbahn (30) eines austrittssicheren Bündchens und dem freien Randabschnitt (330) der austrittssicheren Bündchen-Materialbahn des anderen austrittssicheren Bündchens ist, und W3 ein Abstand zwischen dem die Materialbahn überlappenden Abschnitt (36) eines austrittssicheren Bündchens und dem die Materialbahn überlappenden Abschnitt (36) des anderen austrittssicheren Bündchens ist.

5. Absorbierender Artikel nach Anspruch 4, wobei ein Verhältnis von W1 zu W2 (W1/W2) 1,01 oder mehr und 2,0 oder weniger beträgt.

6. Absorbierender Artikel nach Anspruch 4 oder 5, wobei ein Verhältnis von W3 zu W2 (W3/W2) 1,01 oder mehr und 1,9 oder weniger beträgt.

7. Absorbierender Artikel gemäß Anspruch 1 oder 2, wobei sich der erste Abschnitt (33) in der seitlichen Richtung (Y) außerhalb des zweiten Abschnitts (34) befindet.

8. Absorbierender Artikel gemäß Anspruch 7, wobei der absorbierende Artikel in einem natürlichen Zustand W2 > W1 und W2 > W3 erfüllt, wobei W1 ein Abstand zwischen dem Basisabschnitt (31) eines austrittssicheren Bündchens und dem Basisabschnitt (31) des anderen austrittssicheren Bündchens des Paars austrittssicherer Bündchen (3) ist, W2 ein Abstand zwischen dem freien Randabschnitt (330) der austrittssicheren Bündchen-Materialbahn (30) eines austrittssicheren Bündchens und dem freien Randabschnitt (330) der austrittssicheren Bündchen-Materialbahn (30) des anderen austrittssicheren Bündchens ist, und W3 ist ein Abstand zwischen dem die Materialbahn überlappenden Abschnitt (36) eines austrittssicheren Bündchens und dem die Materialbahn überlappenden Abschnitt (36) des anderen austrittssicheren Bündchens ist.

9. Absorbierender Artikel nach Anspruch 8, wobei ein Verhältnis von W1 zu W2 (W1/W2) 0,6 oder mehr und 0,99 oder weniger beträgt.

10. Absorbierender Artikel nach Anspruch 8 oder 9, wobei ein Verhältnis von W3 zu W2 (W3/W2) 0,55 oder mehr und 0,95 oder weniger beträgt.

11. Absorbierender Artikel nach einem der Ansprüche 1 bis 10, wobei ein Verhältnis der Spannung des innersten elastischen Elements (37a) zu der Spannung des äußersten elastischen Elements (37b) 1,01 oder mehr und 3 oder weniger beträgt.

12. Absorbierender Artikel nach einem der Ansprüche 1 bis 11, wobei die Spannung des innersten elastischen Elements (37b) 0,03 N oder mehr und 0,24 N oder weniger beträgt.

13. Absorbierender Artikel nach einem der Ansprüche 1 bis 12, wobei die Spannung des äußersten elastischen Elements (37b) 0,02 N oder mehr und 0,2 N oder weniger beträgt.

14. Absorbierender Artikel nach einem der Ansprüche 1 bis 13, wobei der Verbindungsabschnitt (35) ein verschweißter Abschnitt ist, an dem der erste Abschnitt (33) mit dem zweiten Abschnitt (34) verschweißt ist, und sich eine von dem ersten Abschnitt (33) aus gesehene Fläche des verschweißten Abschnitts von einer von dem zweiten Abschnitt (34) aus gesehenen Fläche des verschweißten Abschnitts unterscheidet .

## Revendications

1. Article absorbant présentant une direction longitudinale (X) correspondant à une direction avant-arrière d'un porteur et une direction latérale (Y) orthogonale à la direction longitudinale (X), l'article absorbant comprenant :
un ensemble absorbant (2) comportant un élément absorbant (23) ; et
une paire de revers étanches (3) s'étendant dans la direction longitudinale (X) et prévus sur une surface faisant face à la peau de l'ensemble absorbant (2) alors qu'une partie de séparation (39) est intercalée entre eux, dans lequel
chaque revers de la paire de revers étanches (3) comporte une feuille (30) formant revers étanche et présente une partie de base (31) sur laquelle la feuille (30) formant revers étanche est fixée à un autre élément et une partie de soulèvement (32) qui soulève la feuille (30) formant revers étanche vers le porteur depuis la partie de base (31) en tant qu'un point de départ,
la feuille (30) formant revers étanche constituant la partie de soulèvement (32) est pliée le long d'une partie de pliage (30F) s'étendant dans la direction longitudinale (X),
la partie de soulèvement (32) est sectionnée en une première partie (33) depuis la partie de pliage (30F) jusqu'à un bord latéral de la feuille (30) formant revers étanche le long de la direction longitudinale (X) et une seconde partie (34) depuis la partie de pliage (30F) jusqu'à la partie de base (31) et comporte une partie (36) chevauchant la feuille dans laquelle la première partie (33) et la seconde partie (34) se faisant face sont jointes par l'intermédiaire d'une partie jointe (35),
la première partie (33) comporte une partie de bord libre (330), la partie de bord libre (330) n'est fixée à aucun autre élément, et est une partie d'extension de la feuille (30) formant revers étanche s'étendant depuis la partie jointe (35), et
une longueur d'extension L1 de la partie de bord libre (330) depuis la partie jointe (35) n'est pas inférieure à 0,05 fois une longueur L2 depuis la partie jointe (35) jusqu'à la partie de base (31) dans la seconde partie (34), **caractérisé en ce que**
sont disposés, entre la première partie (33) et la seconde (34), une pluralité d'éléments élastiques (37) formant le revers étanche dans la direction latérale (Y), et
un élément élastique le plus à l'intérieur (37a) situé le plus à l'intérieur dans la direction latérale (Y) de la pluralité d'éléments élastiques (37) formant revers étanche présente une contrainte supérieure à un élément élastique le plus à l'extérieur (37b) situé le plus à l'extérieur dans la direction latérale (Y) lorsque l'article absorbant est porté.

2. Article absorbant selon la revendication 1, dans lequel la longueur d'extension L1 de la partie de bord libre (330) depuis la partie jointe (35) n'est pas inférieure à 0,1 fois et n'est pas supérieure à 0,9 fois la longueur L2 depuis la partie jointe (35) jusqu'à la partie de base (31) dans la seconde partie (34).

3. Article absorbant selon la revendication 1 ou 2, dans lequel la première partie (33) est située à l'intérieur de la seconde partie (34) dans la direction latérale (Y).

4. Article absorbant selon la revendication 3, dans lequel l'article absorbant satisfait à W2 < W1 et W2 < W3 dans un état naturel, où W1 est une distance entre la partie de base (31) d'un revers étanche et la partie de base (31) de l'autre revers étanche de la paire de revers étanches (3), W2 est une distance entre la partie de bord libre (330) de la feuille (30) formant revers étanche d'un revers étanche et la partie de bord libre (330) de la feuille (30) formant revers étanche de l'autre revers étanche, et W3 est une distance entre la partie (36) chevauchant la feuille d'un revers étanche et la partie (36) chevauchant la feuille de l'autre revers étanche.

5. Article absorbant selon la revendication 4, dans lequel un rapport de W1 à W2 (W1/W2) est de 1,01 ou plus et de 2,0 ou moins.

6. Article absorbant selon la revendication 4 ou 5, dans lequel un rapport de W3 à W2 (W3/W2) est de 1,01 ou plus et de 1,9 ou moins.

7. Article absorbant selon la revendication 1 ou 2, dans lequel la première partie (33) est située à l'extérieur de la seconde partie (34) dans la direction latérale (Y).

8. Article absorbant selon la revendication 7, dans lequel l'article absorbant satisfait à W2 > W1 et W2 > W3 dans un état naturel, où W1 est une distance entre la partie de base (31) d'un revers étanche et la partie de base (31) de l'autre revers étanche de la paire de revers étanches (3), W2 est une distance entre la partie de bord libre (330) de la feuille (30) formant revers étanche d'un revers étanche et la partie de bord libre (330) de la feuille (30) formant revers étanche de l'autre revers étanche, et W3 est une distance entre la partie (36) chevauchant la feuille d'un revers étanche et la partie (36) chevauchant la feuille de l'autre revers étanche.

9. Article absorbant selon la revendication 8, dans lequel un rapport de W1 à W2 (W1/W2) est de 0,6 ou plus et de 0,99 ou moins.

10. Article absorbant selon la revendication 8 ou 9, dans lequel un rapport de W3 à W2 (W3/W2) est de 0,55 ou plus et de 0,95 ou moins.

11. Article absorbant selon l'une quelconque des revendications 1 à 10, dans lequel un rapport de la contrainte de l'élément élastique le plus à l'intérieur (37a) sur la contrainte de l'élément élastique le plus à l'extérieur (37b) est de 1,01 ou plus et de 3 ou moins.

12. Article absorbant selon l'une quelconque des revendications 1 à 11, dans lequel la contrainte de l'élément élastique le plus à l'intérieur (37b) est de 0,03 N ou plus et de 0,24 N ou moins.

13. Article absorbant selon l'une quelconque des revendications 1 à 12, dans lequel la contrainte de l'élément élastique le plus à l'extérieur (37b) est de 0,02 N ou plus et de 0,2 N ou moins.

14. Article absorbant selon l'une quelconque des revendications 1 à 13, dans lequel la partie jointe (35) est une partie fondue sur laquelle la première partie (33) est fondue avec la seconde partie (34), et une zone de la partie fondue observée depuis la première partie (33) diffère d'une zone de la partie fondue observée depuis la seconde partie (34).
